# EUROPEAN PATENT APPLICATION

(11) **EP 3 407 225 A1**
(43) Date of publication of application: **28.11.2018**
(21) Application number: 17172482.6
(22) Date of filing: 23.05.2017
(51) Int. Cl.: G06F 19/00, A61B 5/11

(54) **REHABILITATION DEVICE AND METHOD**

(71) Applicant: Koninklijke Philips N.V., 5656 AE Eindhoven (NL)
(72) Inventor: JIN, Kim, 5656 AE Eindhoven (NL); WANG, Jim, 5656 AE Eindhoven (NL)
(74) Representative: de Haan, Poul Erik

(57) **Abstract**

A rehabilitation device (100) for a rehabilitation exercise is described. The device comprises a database interface (110) arranged to access a database (50) comprising a plurality of patient profiles. The patient profiles comprise motion information of patients in a rehabilitation exercise and characteristics of the rehabilitation exercise. The device (100) comprises a processor (120) arranged to receive competitor measures comprising characteristics of a rehabilitation exercise arranged for a user. The processor (120) retrieves one or more patient profiles as identified competitor's profiles from the database based on the similarity between the competitor measures in the patient profiles and the competitor measures received, and extracts motion information from each of the competitor's profiles for display during the rehabilitation exercise arranged for the user. By showing what other users are able to achieve during the same exercise, the user is encouraged to perform better. In this way, the user will stay motivated and will recover faster from the injury as compared to the use of prior art devices.

## Description

### FIELD OF THE INVENTION

The invention relates to a rehabilitation device and system for a rehabilitation exercise. The invention further relates to a method of operating a rehabilitation device, and to a computer program product for operating such a rehabilitation device.

### BACKGROUND OF THE INVENTION

The document US2013/123667 A1 describes an apparatus and method for the non-invasive motion tracking to augment patient administered physical therapy via a motion tracking apparatus, a display, and a computing platform coupled to the motion tracking apparatus and the display. The computing platform serves to provide a menu driven interface to the patient, an instruction to the patient, a determination of the patient's motion or action in response to the instruction, a comparison between the instruction to the patient and the determination of the patient's motion or action, and to provide a feedback display to the patient. With real time data input from a sensor suite consisting of a motion tracking or video capture systems, such as a camera array and microphone array, the patient's motion, speech, and other patient characteristics can be accurately tracked and used by the program to provide analysis of performance both longitudinally and in comparison to a predefined motion path to provide pertinent feedback and instruction. The feedback will help the user to do the exercises, but there is a risk that the user will get demotivated after a while if she thinks that she is not making any or sufficient progress.

### SUMMARY OF THE INVENTION

It is an object of the invention to provide device and method to improve the user motivation during a rehabilitation exercise so as to speed up the rehabilitation process.

For this purpose, according to a first aspect of the invention, a rehabilitation device for a rehabilitation exercise is provided, the device comprising a database interface arranged to access a database comprising a plurality of patient profiles, each of the patient profiles comprising motion information of a patient in a rehabilitation exercise and characteristics of the rehabilitation exercise. A processor arranged to receive competitor measures comprising characteristics of a rehabilitation exercise arranged for a user, to retrieve one or more patient profiles as identified competitor's profiles from the database based on the similarity between the competitor measures in the patient profiles and the competitor measures received, and to extract motion information from each of the competitor's profiles for display during the rehabilitation exercise arranged for the user.

For this purpose, according to a second aspect of the invention, a rehabilitation system for a rehabilitation exercise is provided comprising a database comprising a plurality of patient profiles, each of the patient profiles comprising motion information of a patient in a rehabilitation exercise and characteristics of the rehabilitation exercise, and a number of rehabilation devices as described above.

For this purpose, according to a third aspect of the invention, a method of operating a rehabilitation device is provided, the method comprising:
- accessing a database comprising a plurality of patient profiles, each of the patient profiles comprising motion information of a patient in a rehabilitation exercise and characteristics of the rehabilitation exercise;
- receiving competitor measures comprising characteristics of a rehabilitation exercise arranged for a user;
- retrieving one or more patient profiles as identified competitor's profiles from the database based on the similarity between the competitor measures in the patient profiles and the competitor measures received, and
- extracting motion information from each of the competitor's profiles for display during the rehabilitation exercise arranged for the user.

For this purpose, according to a fourth aspect of the invention, a computer program product for operating a rehabilitation device, which program is operative to cause a processor to perform the method as described above.

By showing what other users are able to achieve during the same exercise, the user is encouraged to perform better. In this way, the user will stay motivated and will recover faster from the injury as compared to the use of prior art devices.

In an embodiment of the rehabilitation device, the competitor measures further comprise the motion information of the user in the rehabilitation exercise. By using motion information of the user, possibly converted into a preformance score, a competitor's profile closer to the condition of the user is used to select competitors. This will result in a more competitive type of exercise, and will increase the motivation of the user.

In an embodiment, the motion information of the user or the patient in the rehabilitation exercise comprises at least one of:
- a motion trajectory of the user or the patient in the rehabilitation exercise;
- a performance level of the user or the patient derived from the motion trajectory of the user in the rehabilitation exercise;
- an abnormal pattern of the motion trajectory of the user or the patient;
- a motion capability level of the user or the patient;
- motion speed of of the user or the patient in the rehabilitation exercise.

The advantage of gathering suitable motion information of the user of the device, is that suitable measures can be used to find the most relevant competitors of the user by comparing the measures of the user with te measures of the profiles stored in the database. On the other hand, the motion information extracted from competitor's profile will be displayed to the users during the exercises to motivate the user. The motion information in the competitor measures received is not necessarily to be the same that is displayed later to the user during the exercise.

In an embodiment, the processor is arranged to display the guidance during the rehabilitation exercise. The guidance may comprise a target motion trajectory, and/or the motion trajectory of the user in the rehabilitation exercise. By showing the target trajector, the user will be guided and stimulated to follow an optimal trajectory. The motion trajectory of the user can be displayed to give direct visual feedback to the user.

In an embodiment, the characteristics of the rehabilitation exercise comprises at least one of a target body part of the rehabilitation exercise, contents of the rehabilitation exercise, an identifier of the rehabilitation exercise, and a presciption source of the rehabilitation treatment comprising the rehabilitation exercise. These characteristics of the exercise can be used as measures to filter the patient profiles in a fast and adequat way. The contents of the rehabilitation exercise may refer to the target trajectory and according repetition times, or anything that is available in an exercise prescription.

In an embodiment, the processor is arranged to retrieve a first patient profile with a performance level below the performance level of the user, and a second patient profile with a performance level above the performance level of the user. This may be advantageous because the user will understand her performance level by showing a better and a worse performing competitor, and will be more motivated to stay away from the latter while wanting to beat the former.

In an embodiment, the processor is arranged to compare a target trajectory to at least one of the competitor motion trajectories and to determine one or more attention points on the target trajectory where a deviation between the target trajectory and the competitor motion trajectories exceeds a deviation threshold. The indication of attention points will warn the user to concentrate on difficult parts of the exercise. Parts that were difficult for the competitors is most probably also difficult for the user.

In an embodiment, the processor is arranged to determine a speed of a body part of the user and to determine one or more attention points on the motion trajectory of the user where the speed of the body part previously exceeded a speed threshold. By recording the speed during the exercise, and comparing the speed with a threshold, the device is able to give feedback to the user at moments/points where the user has to move his body part slower, even if this is difficult. This will decrease the recovery period.

In an embodiment, the motion information of the competitor profiles comprises speed information relating to a speed of a part of the body of the competitor during the rehabilitation exercise. The processor can compare the speed of the body part of the competitors to a predetermined competitor speed threshold, and can determine one or more attention points on the motion trajectory of the user where the speed of the body part of at least one of the compatitors exceeds the competitor speed threshold. By recording the speed during the exercise for all the patients, and comparing the speed with a threshold, the device is able to give feedback to the user at moments/points where the competitor have failed, had problems, due to the attentoin points, the user is able to focus on such points. This will decrease the recovery period.

In an embodiment, the processor is arranged to generate an indication of the attention points for display. The user will look at the display during the exercise. So by also displaying the attention points along the trajectory, she will focus at the right time to do his best especially at attentions points.

In an embodiment, the patient profiles each comprise a history of motion trajectories with one or more motion trajectories and associated time stamps, wherein the processor is arranged to display one or more motion trajectories of the selected patient profiles that best resemble a freshness level of the user. By comparing the results of the user to the results of competitors with similar freshness levels, the user will not be discouraged because she will understand that the comparison is fair.

In an embodiment, the device further comprises a communication interface for communication to other rehabilitation devices, wherein the processor is arranged to set up a link between the rehabilitation device and one or more of the other rehabilitation devices, for communication between users of the devices. By giving the user the ability to communicate with other users, she will be more motivated since it is more fun and the other users might be able to encourage the user by sending encouraging messages and/or speaking to her.

Further preferred embodiments of the device and method according to the invention are given in the appended claims, disclosure of which is incorporated herein by reference.

### BRIEF DESCRIPTION OF THE DRAWINGS

These and other aspects of the invention will be apparent from and elucidated further with reference to the embodiments described by way of example in the following description and with reference to the accompanying drawings, in which
Figure 1 shows a rehabilitation device for a rehabilitation exercise according to an embodiment;
Figure 2 schematically shows an example of a picture displayed on the display;
Figure 3 shows a rehabilitation system for a rehabilitation exercise according to an embodiment of the invention,
Figure 4 is a flow chart of a method 400 of operating a rehabilitation device according to an embodiment of the invention, and
Figure 5 shows an embodiment of a computer readable medium storing instructions for the computer to perform the method of Figure 4.

The figures are purely diagrammatic and not drawn to scale. In the Figures, elements which correspond to elements already described may have the same reference numerals.

### DETAILED DESCRIPTION OF EMBODIMENTS

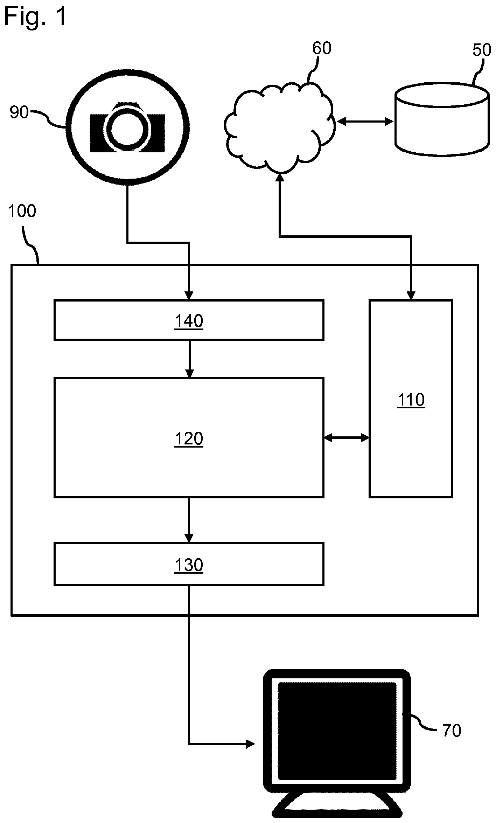

Figure 1 shows a rehabilitation device 100 for a rehabilitation exercise according to an embodiment. The device 100 comprises a database interface 110 arranged to access a database 50, optionally via a communication network 60, such as a LAN or WAN. The device 100 further comprises a processor 120. In this example, the device 100 also comprises an output interface 130 for data communication of the processor to a display 70. In this embodiment, the device 100 also comprises an input interface 140 for receiving image data from an imaging device, such as a camera 90.

The rehabilitation device 100 may be used by a person, such as a patient recovering from a stroke, to do rehabilitation exercises. The person will see instructions on the display 70, optionally with images of herself recorded by the camera 90.

The processor 100 may be arranged to guide the user by displaying, during the rehabilitation exercise, a target motion trajectory and optionally also a motion trajectory of the user in the rehabilitation exercise. By showing the target trajectory on the dispay 70, the user will be guided and stimulated to follow an optimal trajectory. The motion trajectory of the user can be displayed to give direct visual feedback to the user. If both the target trajectory and the motion trajectory are shown, the user will get information on how well she is performing, which will stimulate her in doing her best. This will help in speeding up the rehabilitation proces.

To further stimulate or motivate the person doing the exercise, information about other patients doing, or having done, in the rehabilitation exercises is used by the device. For that purpose, the database may comprise a plurality of patient profiles, each of the patient profiles comprising motion information of a patient in a rehabilitation exercise and characteristics of the rehabilitation exercise. The processor 120 may be arranged to receive competitor measures comprising characteristics of a rehabilitation exercise arranged for a user. These characteristics of the rehabilitation exercise may be received from the user via a User Interface, such as a keyboard or GUI (not shown in Figure 1) or via the camera 90, or from a memory (not shown). A typical example of such characteristics is an exercise identification code, identifying the rehabilitation exercise.

The processor 120 is arranged to retrieve one or more patient profiles as identified competitor's profiles from the database 50 based on the similarity between the competitor measures in the patient profiles and the competitor measures received. The processor 120 will extract motion information from each of the competitor's profiles for display during the rehabilitation exercise arranged for the user. The competitor measures received may comprise the motion information of the user in the rehabilitation exercise

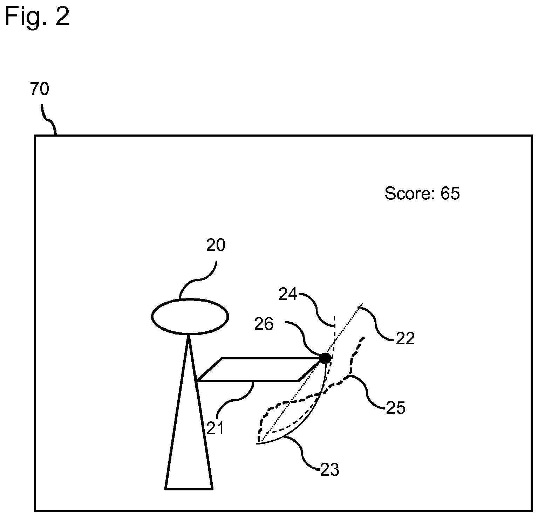

Figure 2 schematically shows an example of a picture displayed on the display 70 to discuss the invention in more detail. In Figure 2 the user 20 is displayed. The user 20 is moving her arm 21 during a rehabilitation exercise. She looks at the display 70 and a line 22 representing a target trajectory 22. The processor 120 may further be configured to control the display to output a figure to represent the body(or part of her body) of the user at the display 70. The target trajectory 22 can be indicated by a coloured line, a dashed line, or any other suitable line recognizable for the user.

The movement of her arm is monitored by the camera 90 and a line 23 is displayed on the display 70 representing a trajectory generated based on the movement of her hand during the exercise. By looking at the two lines 22 and 23, she will realize that she moves her arm in a suboptimal way and that improvement is possible. She will try to do better, thereby guided by the target trajectory 22.

To further motivate the user, motion information from other users, also referred to as competitors, is diplayed on the display 70 during the rehabilitation exercise. In this example, a line 24 is shown indicating a trajectory 24 generated by a competitor A, and a further line 25 is shown indicating a trajectory 25 generated by a competitor B. The trajectory 24 or the trajectory 25 may be the latest trajectory or the trajactory generated based on historical records, for example, at an average level. In this example, a processor 120 has retrieved two users which perfomed the same exercise as the current user, wherein motion information of a relatively advanced user, i.e. user A, and of a relatively beginner, i.e. user B, is retrieved from the database 50 and used for display.

By showing what other users are able to achieve during the same exercise, the user is encouraged to perform better. In this way, the user will stay motivated and will recover faster from the injury as compared to the use of prior art devices. Optionally, a score is calculated and displayed on the screen. The score can be calculated using any one of the trajectories discussed, as will be appreciated by the skilled person. For example a score of 100 may indicate that the user followed the target trajectory 22 correctly, where a lower score will indicate that some deviation from the targer trajectory 22 is detected. Instead of the target trajectory 23, the trajectories of the competitors may be used to calculate the score.

In an embodiment, the processor 120 is arranged to compare a target trajectory to at least one of the competitor motion trajectories 24, 25 and to determine one or more attention points on the target trajectory 22 where a deviation between the target trajectory and the competitor motion trajectories exceeds a deviation threshold. In Figure 2, an example of such an attention point is shown, see point 26, which devicates the most from the target trajectory 22 in the trajectory 25 of competitor B. The indication of such an attention point 26 will warn the user to concentrate on difficult parts of the exercise. A part that was difficult for the competitors is most probably also difficult for the user.

Instead of trajectories, other motion information can be used to calculate the score. For example, speed of the hand may be compared to the optimal speed determined by a trainer who pre-programmed the device 100. For example, the processor 120 may be arranged to determine a speed of a body part of the user and to determine one or more attention points on the motion trajectory of the user where the speed of the body part previously exceeded a speed threshold. By recording the speed during the exercise, and comparing the speed with a threshold, the device 100 is able to give feedback to the user at moments/points where the user has to move his body part slower, even if this is difficult. This will further decrease the recovery period.

The motion information of the competitor profiles may comprise speed information relating to a speed of a part of the body of the competitor during the rehabilitation exercise. The processor 120 may then be arranged to compare the speed of the body part of the competitors to a predetermined competitor speed threshold, and to determine one or more attention points on the motion trajectory of the user where the speed of the body part of at least one of the compatitors exceeds the competitor speed threshold. By recording the speed during the exercise for all the patients, and comparing the speed with a threshold, the device is able to give feedback to the user at moments/points where the competitor have failed, had problems, due to the attentoin points, the user is able to focus on such points. This will also decrease the recovery period.

The current user may configure her preference on competitor selection before the training. Then the processor 100 may initiate a search through the database of patient profiles to find qualified competitors, e.g. other patients with the same capability and retrieve the historical/instantaneous motion trajectories for comparison. Such comparison may also provide useful information to assist the patient to excel the training, e.g. the point where other patient fails. By using the motion information of the user to select competitors, a competitor's profile closer to the condition of the user can be selected and used for display. This will result in a more competitive way of exercising.

In an embodiment, the device 100 further comprises a communication interface for communication to other rehabilitation devices. The communication interface may be implemented using a graphical user interface (GUI) displayed on the display 70 wherein the user may use a keyboard to enter commands or messages, or use a microphone to enter commmands or messages. The processor may be arranged to set up a link between the rehabilitation device 100 and one or more of the other rehabilitation devices, for communication between users of the devices. By giving the user the ability to communicate with other users, she will be more motivated since it is more fun and the other users might be able to encourage the user by sending encouraging messages or speaking to her.

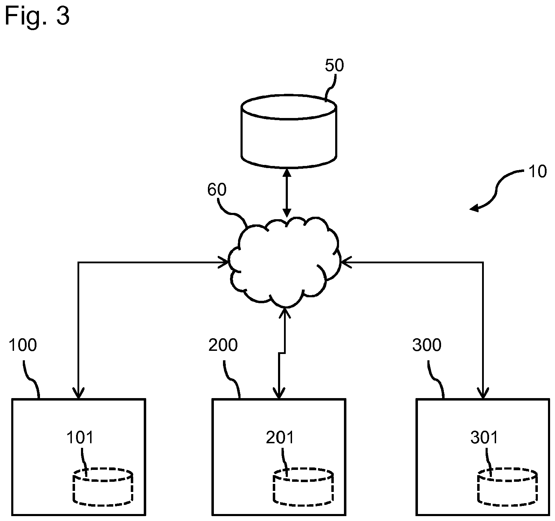

Figure 3 shows a rehabilitation system 10 for a rehabilitation exercise according to an embodiment of the invention. The system 10 comprises a database 50 comprising a plurality of patient profiles, each of the patient profiles comprising motion information of a patient in a rehabilitation exercise and characteristics of the rehabilitation exercise. The system further comprises a number of rehabilation devices 100, 200 and 300. The device 100, 200 and 300 may be devices as described with reference to Figures 1 and 2.

In Figure 3 the database 50 is a central database accessible via a communication network 60. Alternatively, or additionally, local databases 101, 102, 103 may be arranged in the devices 100, 200 and 300. The local databases 101, 201, 301 may store patient profiles of the user(s) using the associated device, i.e. device 100, 200, 300 respectively. It is noted that the patient profiles may be stored locally and centrally, wherein the devices are arranged to synchronize the content of the local databases with the central database or between the local databases 101, 201, 301.

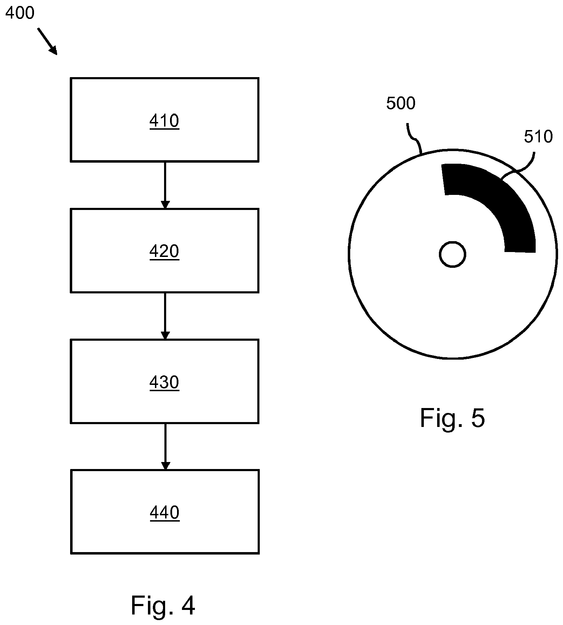

Figure 4 is a flow chart of a method 400 of operating a rehabilitation device according to an embodiment of the invention. It is noted that the method 400 may, but does not need to, correspond to an operation of the system 100 as described with reference to Figure 1 and others.

The method 400 comprises in an operation titled "ACCESSING A DATABASE", accessing 410 a database comprising a plurality of patient profiles, each of the patient profiles comprising motion information of a patient in a rehabilitation exercise and characteristics of the rehabilitation exercise. The method further comprises, in an operation entitled "RECEIVING COMPETITOR MEASURES", receiving 420 competitor measures comprising characteristics of a rehabilitation exercise arranged for a user. The method 400 further comprises, in an operation titled "RETRIEVING ONE OR MORE PATIENT PROFILES", retrieving 430 one or more patient profiles as identified competitor's profiles from the database based on the similarity between the competitor measures in the patient profiles and the competitor measures received. The method 400 further comprises, in an operation titled "EXTRACTING MOTION INFORMATION", extracting 440 motion information from each of the competitor's profiles for display during the rehabilitation exercise arranged for the user.

It will be appreciated that the above operation may be performed in any suitable order, e.g., consecutively, simultaneously, or a combination thereof, subject to, where applicable, a particular order being necessitated, e.g., by input/output relations.

The method 400 may be implemented on a computer as a computer implemented method, as dedicated hardware, or as a combination of both. As also illustrated in Figure 5, instructions for the computer, e.g., executable code, may be stored on a computer readable medium 500, e.g., in the form of a series 510 of machine readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, servers, online software, etc. Figure 5 shows an optical disc 500.

Above a rehabilitation device for a rehabilitation exercise is described. The device comprises a database interface arranged to access a database comprising a plurality of patient profiles. The patient profiles comprise motion information of patients in a rehabilitation exercise and characteristics of the rehabilitation exercise. The device comprises a processor arranged to receive competitor measures comprising characteristics of a rehabilitation exercise arranged for a user. The processor retrieves one or more patient profiles as identified competitor's profiles from the database based on the similarity between the competitor measures in the patient profiles and the competitor measures received, and extracts motion information from each of the competitor's profiles for display during the rehabilitation exercise arranged for the user.

It will be appreciated that the above description for clarity has described embodiments of the invention with reference to different functional units and processors. However, it will be apparent that any suitable distribution of functionality between different functional units or processors may be used without deviating from the invention. For example, functionality illustrated to be performed by separate units, processors or controllers may be performed by the same processor or controllers. Hence, references to specific functional units are only to be seen as references to suitable means for providing the described functionality rather than indicative of a strict logical or physical structure or organization. The invention can be implemented in any suitable form including hardware, software, firmware or any combination of these.

It is noted, that in this document the word 'comprising' does not exclude the presence of other elements or steps than those listed and the word 'a' or 'an' preceding an element does not exclude the presence of a plurality of such elements, that any reference signs do not limit the scope of the claims, that the invention may be implemented by means of both hardware and software, and that several 'means' or 'units' may be represented by the same item of hardware or software, and a processor may fulfill the function of one or more units, possibly in cooperation with hardware elements. Further, the invention is not limited to the embodiments, and the invention lies in each and every novel feature or combination of features described above or recited in mutually different dependent claims.

## Claims

1. A rehabilitation device (100) for a rehabilitation exercise, the device comprising:
- a database interface (110) arranged to access a database (50) comprising a plurality of patient profiles, each of the patient profiles comprising motion information of a patient in a rehabilitation exercise and characteristics of the rehabilitation exercise;
- a processor (120) arranged to:
- receive competitor measures comprising characteristics of a rehabilitation exercise arranged for a user;
- retrieve one or more patient profiles as identified competitor's profiles from the database based on the similarity between the competitor measures in the patient profiles and the competitor measures received, and
- extract motion information from each of the competitor's profiles for display during the rehabilitation exercise arranged for the user.

2. The rehabilitation device according to claim 1, wherein the competitor measures further comprise the motion information of the user in the rehabilitation exercise.

3. The rehabilitation device according to claim 1, wherein the motion information of the user or the patient in the rehabilitation exercise comprises at least one of:
- a motion trajectory of the user or the patient in the rehabilitation exercise;
- a performance level of the user or the patient derived from the motion trajectory of the user in the rehabilitation exercise;
- an abnormal pattern of the motion trajectory of the user or the patient;
- a motion capability level of the user or the patient;
- motion speed of of the user or the patient in the rehabilitation exercise.

4. The rehabilitation device according to claim 2, wherein the processor is arranged to display the guidance during the rehabilitation exercise, the guidance comprising at least one of:
- a target motion trajectory;
- the motion trajectory of the user in the rehabilitation exercise.

5. The rehabilitation device according to any one of the preceding claims, wherein the characteristics of the rehabilitation exercise comprises at least one of:
- a target body part of the rehabilitation exercise;
- contents of the rehabilitation exercise;
- an identifier of the rehabilitation exercise;
- a presciption source of the rehabilitation treatment comprising the rehabilitation exercise.

6. The rehabilitation device according to any one of claims 2-4, wherein the processor is arranged to retrieve a first patient profile with a performance level below the performance level of the user, and a second patient profile with a performance level above the performance level of the user.

7. The rehabilitation device according to any one of the preceding claims, wherein the processor is arranged to compare a target trajectory to at least one of the competitor motion trajectories and to determine one or more attention points on the target trajectory where a deviation between the target trajectory and the competitor motion trajectories exceeds a deviation threshold.

8. The rehabilitation device according to any one of the preceding claims 1-6, wherein the processor is arranged to determine a speed of a body part of the user and to determine one or more attention points on the motion trajectory of the user where the speed of the body part previously exceeded a speed threshold.

9. The rehabilitation device according to any one of the preceding claims 1-6, wherein the motion information of the competitor profiles comprises speed information relating to a speed of a part of the body of the competitor during the rehabilitation exercise, and wherein the processor is arranged to:
- compare the speed of the body part of the competitors to a predetermined competitor speed threshold, and
- determine one or more attention points on the motion trajectory of the user where the speed of the body part of at least one of the compatitors exceeds the competitor speed threshold.

10. The rehabilitation device according to proceeding claims 7-9, wherein the processor is arranged to generate an indication of the attention points for display.

11. The rehabilitation device according to any one of the preceding claims, wherein the one or more patient profiles each comprise a history of motion trajectories with one or more motion trajectories and associated time stamps, wherein the processor is arranged to display one or more motion trajectories of the selected patient profiles that best resemble a freshness level of the user.

12. The rehabilitation device according to any one of the preceding claims, the device further comprising a communication interface for communication to other rehabilitation devices, wherein the processor is arranged to set up a link between the rehabilitation device and one or more of the other rehabilitation devices, for communication between users of the devices.

13. A rehabilitation system (10) for a rehabilitation exercise, the system comprising:
- a database (50) comprising a plurality of patient profiles, each of the patient profiles comprising motion information of a patient in a rehabilitation exercise and characteristics of the rehabilitation exercise;
- a number of rehabilation devices (100, 200, 300) according to any one of the preceeding claims.

14. A method of operating a rehabilitation device, the method comprising:
- accessing a database comprising a plurality of patient profiles, each of the patient profiles comprising motion information of a patient in a rehabilitation exercise and characteristics of the rehabilitation exercise;
- receiving competitor measures comprising characteristics of a rehabilitation exercise arranged for a user;
- retrieving one or more patient profiles as identified competitor's profiles from the database based on the similarity between the competitor measures in the patient profiles and the competitor measures received, and
- extracting motion information from each of the competitor's profiles for display during the rehabilitation exercise arranged for the user.

15. Computer program product for operating a rehabilitation device, which program is operative to cause a processor to perform the method as claimed in claim 14.
